# EUROPÄISCHE PATENTANMELDUNG

(11) **EP 4 079 336 A1**
(43) Veröffentlichungstag der Anmeldung: **26.10.2022**
(21) Anmeldenummer: 22020181.8
(22) Anmeldetag: 19.04.2022
(51) Int. Cl.: A61L 2/26

(54) **GRIFF FÜR EINEN STERILISATIONSBEHÄLTER**

(30) Priorität: 21.04.2021 DE 202021102136 U
(71) Anmelder: Ermis Medizintechnik e.K., 78532 Tuttlingen (DE)
(72) Erfinder: ERMIS, Mehmet, 78532 Tuttlingen (DE)
(74) Vertreter: Straub, Bernd

(57) **Zusammenfassung**

Der erfindungsgemäße Griff 4 für einen Sterilisationsbehälter 1 ist U-förmig mit zwei Schenkeln 41 und einem als horizontaler Steg 42, ein Halteelement bildend und die beiden Schenkel 41 miteinander verbindet, ausgebildet. Der Griff 4 ist dabei mittels einer Befestigung am Sterilisationsbehälter 1 drehbar befestigt und die Befestigung weist wenigstens einen mittels einer Feder 44 federnd gelagerten Stift 43 auf, der in eine Ausnehmung 31 am Sterilisationsbehälter 1 eingreifen, ein Drehlager bilden und aus der Ausnehmung 31 entgegen der Federkraft durch die Feder 44 entnommen werden kann.

Dadurch ist es möglich, dass auf einfache und verlässliche Weise bei Bedarf der Griff 4 durch Betätigung der federnden Befestigung entnommen beziehungsweise ausgetauscht oder mit dem Sterilisationsbehälter 1 verbunden werden kann. Dabei kann die Feder 44 sowohl als Zugfeder oder als Druckfeder realisiert sein und den Stift 43 in der Ausnehmung 31 am Sterilisationsbehälter 1 federnd halten.

## Beschreibung

### TECHNISCHES GEBIET

Die Erfindung betrifft einen Griff für einen Sterilisationsbehälter.

### STAND DER TECHNIK

Aus der Deutschen Offenlegungsschrift DE 197 55 532 A1 ist ein Sterilcontainer für medizinische Zwecke mit einem wannenförmigen Unterteil und mit einem auf dieses Unterteil aufsetzbaren und abdichtenden Deckel bekannt. Dabei ist der Deckel durch einen Verschluss gegen das Unterteil spannbar ausgebildet. Der Verschluss kann zwischen einer Offenstellung und einer Schließstellung bewegt werden und stellt eine schwenkbare Klappe mit einem Rastvorsprung und einer Rastnase dar. Will man den Sterilcontainer anheben, so muss man entweder das wannenförmige Unterteil umgreifen und den Sterilcontainer anheben oder die durch den Verschluss gebildeten Vorsprünge als Angriffspunkt nutzen. Dieser Sterilcontainer erweist sich in der Handhabung als sehr unbequem.

Aus der Offenlegungsschrift DE 197 23 857 A1 und aus dem Gebrauchsmuster DE 20 2017 102 441 U1 sind Sterilcontainer für medizinische Zwecke bekannt, die ein wannenartiges Unterteil und einen darauf aufsetzbaren Deckel zeigen. An Seitenwänden des Unterteils sind Behälterkennzeichnungsvorrichtungen angeordnet, die eine Grundplatte zur Aufnahme von Etiketten aufweisen. In diese Grundplatte ist ein schwenkbarer Traggriff dauerhaft eingebracht, der die Grundplatte weitgehend umschließt und so nach außen verschwenkt werden kann, dass der Traggriff zum Tragen des Sterilcontainer verwendet werden kann. Dadurch ist die Handhabung dieser Sterilcontainer merklich verbessert.

Weiterhin ist aus dem Gebrauchsmuster DE 20 2014 105 915 U1 ein Sterilisationsbehälter für medizinische Zwecke mit einem Unterteil und einem Deckel bekannt, wobei ein Verschluss dafür sorgt, dass der Deckel mit dem wannenförmigen Unterteil des Sterilisationsbehälters fest verschließbar ist. Er weist einen mehrteiligen Griff auf, welcher zum Transport geeignet ist. Dabei ist der Griff mit Lagerelementen an dem wannenförmigen Unterteil drehbar befestigt. Die Lagerelemente sind dabei als Madenschrauben realisiert. Dadurch ist die Möglichkeit eines Auswechselns des Griffes ermöglicht.

### BESCHREIBUNG DER ERFINDUNG

Der Erfindung liegt die Aufgabe zu Grunde, einen gegenüber dem Stand der Technik verbesserten Griff für einen medizinischen Sterilisationsbehälter anzugeben, der in seiner Handhabung besonders vorteilhaft ist.

Die Aufgabe wird erfindungsgemäß mit einen Griff für einen medizinischen Sterilisationsbehälter gelöst, welcher die im Anspruch 1 angegebenen Merkmale aufweist.

Vorteilhafte Ausgestaltungen der Erfindung sind Gegenstand der Unteransprüche.

Der erfindungsgemäße Griff für einen Sterilisationsbehälter ist U-förmig mit zwei Schenkeln und einem als horizontaler Steg, ein Halteelement bildend und die beiden Schenkel miteinander verbindend, ausgebildet. Der Griff ist dabei mittels einer Befestigung am Sterilisationsbehälter drehbar befestigt und die Befestigung weist wenigstens einen mittels einer Feder federnd gelagerten Stift auf, der in eine Ausnehmung am Sterilisationsbehälter eingreifen, ein Drehlager bilden und aus der Ausnehmung entgegen der Federkraft durch die Feder entnommen werden kann.

Dadurch ist es möglich, dass auf sehr einfache und verlässliche Weise bei Bedarf der Griff durch Betätigung der federnden Befestigung entnommen beziehungsweise ausgetauscht oder mit dem Sterilisationsbehälter verbunden werden kann. Dabei kann die Feder sowohl als Zugfeder als auch als Druckfehler realisiert sein und den Stift in der Ausnehmung am Sterilisationsbehälter federnd halten.

Dabei kann die Feder den Stift federnd in Richtung der Ausnehmung drücken, so dass durch eine Bewegung des Stiftes, der ein Drehlager für den Griff definiert, entgegen der Federkraft aus der Ausnehmung heraus bewegt und dadurch die Verbindung zwischen dem Stift des Griffes und der Ausnehmung gelöst und dadurch der Griff entnommen werden kann. In entsprechender Weise kann umgekehrt der Griff wieder mit der Ausnehmung und damit mit dem eigentlichen Sterilisationsbehälter verbunden werden.

Darüber hinaus hat es sich besonders bewährt, den Griff für einen Sterilisationsbehälter dahingehend weiterzubilden, dass die Befestigung mehrere federnd gelagerte Stifte aufweist, die jeweils in eine Ausnehmung am Sterilisationsbehälter eingreifen können und ein Drehlager bilden können und aus der Ausnehmung entgegen der Federkraft einer Feder entnommen werden können. Dabei sind bevorzugt mehrere der Stifte so orientiert, dass sie eine gemeinsame Drehachse für den Griff festlegen und insbesondere so orientiert sind, dass ihre Federkraft in entgegengesetzte Richtungen zeigt. Dadurch ist ein sehr sicheres Drehlager und eine ausgesprochen einfache Handhabung insbesondere beim Austausch gegeben, zumal in diesem Fall sowohl beide oder mehrere federnd gelagerte Stifte entgegen der Federkraft einer Feder aus der Ausnehmung entnommen werden können oder nur ein Teil davon insbesondere nur ein einziger Stift entnommen werden kann und dennoch der Griff von dem Sterilisationsbehälter gelöst und gegebenenfalls ausgetauscht werden kann.

Als besonders bevorzugter Griff für einen Sterilisationsbehälter hat sich ein Griff gezeigt, bei dem wenigstens ein federnd gelagerter Stift eine verjüngende Spitze aufweist, welche geeignet ist, in die Ausnehmung einzugreifen. Durch diese Ausbildung des Stiftes wird ein zentrierendes, selbst integrierendes Einführen des Stiftes in die Ausnehmung an dem Sterilisationsbehälter besonders sicher und einfach ermöglicht.

Weiterhin hat es sich zusätzlich bewährt, den Griff für einen Sterilisationsbehälter so weiterzubilden, dass wenigstens ein insbesondere alle federnd gelagerten Stifte ein Griffelement aufweisen, das den Stift lateral überragt. Insbesondere ist dabei das Griffelement am Stift an einem Ende des Stiftes angeordnet und bildet einen Kopf beziehungsweise einen zylinderförmigen Kopf. Durch diese Ausbildung des Griffelementes gelingt es, den Stift auch unter schwierigen Umständen sehr sicher zu ergreifen und dadurch die Handhabung des Griffes für einen medizinischen Sterilisationsbehälter sowohl sicher als auch einfach zu ermöglichen.

Eine besonders bevorzugte und besonders vorteilhaft handhabbare Ausbildung der Erfindung zeigt einen Griff für einen Sterilisationsbehälter, bei dem wenigstens ein Griffelement oder Kopf am Ende des Stifts im Randbereich des Schenkels insbesondere im dem Steg abgewandten Randbereich des Schenkels angeordnet ist.

Darüber hinaus hat es sich als besonders vorteilhaft erwiesen, den Griff für einen Sterilisationsbehälter so weiterzubilden, dass wenigstens ein Stift in einer einen Schenkel querenden Aufnahme geführt und insbesondere gehalten ist. Durch diese Führung ist eine einfache Handhabung des Stiftes im besonderen Maße gegeben, insbesondere dann, wenn mehrere Stifte und deren zugehörigen Aufnahmen eine gemeinsame Drehachse bilden.

Nach einer bevorzugten Weiterbildung der Erfindung ist die Aufnahme für den Stift im dem Steg abgewandten Endbereich des Schenkels des Griffs für einen Sterilisationsbehälter angeordnet. Dies sorgt für einen großen Abstand zwischen der Drehachse, die durch die Aufnahme beziehungsweise den Stift definiert ist, zu dem Steg und dadurch zu einem besonders gut handhabbaren Griff.

Vorzugsweise ist der Griff für einen Sterilisationsbehälter so weitergebildet, dass wenigstens eine Aufnahme einen Stift umschließt und ein Gleitlager für einen darin angeordneten Stift bildet. Diese Aufnahme für den Stift bildet sowohl ein Drehlager für den darin gelagerten Stift wie auch ein Gleitlager für den in Längsrichtung verschieblichen, federbewehrten Stift. Diese Konstruktion erweist sich als sehr kompakt und sehr gut handhabbar.

Besonders nützlich hat es sich dabei erwiesen, wenigstens eine Hülse in oder an einem Schenkel des Griffes für einen Sterilisationsbehälter vorzusehen, die eine Führung und/oder ein Gleitlager für einen darin angeordneten Stift bildet. Durch das Vorsehen einer zusätzlichen Hülse ist es möglich, eine besonders effiziente insbesondere reibungsarme Führung beziehungsweise Lagerung des Stiftes in der Hülse, die als Aufnahme für den Stift dient, zu schaffen. Dabei wird die Hülse bevorzugt mittels Presspassung, durch Einschrauben oder Einrasten oder auch durch ein Kleben in einen Stift realisiert und dadurch eine sichere und dauerhafte Positionierung der Hülse gewährleistet.

Auch hat es sich bewährt, den Griff für einen Sterilisationsbehälter so auszubilden, dass die Hülse in der Aufnahme angeordnet ist und insbesondere die Aufnahme abschließt. Damit ist eine besonders effiziente, reibungsarme und verlässliche Lagerung des Stiftes in der Hülse, die die Aufnahme des Stiftes bildet, geschaffen.

Auch hat es sich bewährt, den Griff für einen Sterilisationsbehälter so auszubilden, dass wenigstens eine Feder zwischen dem Stift und der Aufnahme, die in dem Schenkel angeordnet ist und den Stift verschieblich umschließt, angeordnet ist und diese federnd miteinander verbindet. Dabei bilden der Stift mit seinen Befestigungspunkt für die Feder und die Aufnahme in dem Schenkel mit ihrem Befestigungspunkt für die Feder die Widerlager für die dazwischen angeordnete Feder. Diese Feder fördert den Stift durch die Federkraft in Richtung der Ausnehmung zur Bildung einer Befestigung und eines Drehlagers für den Griff. Dabei kann die Feder als Druckfeder oder als Zugfeder ausgebildet sein.

Eine bevorzugte Weiterbildung des erfindungsgemäßen Griffes für einen Sterilisationsbehälter zeigt wenigstens einen Stift mit wenigstens einem Vorsprung oder wenigstens einem ringförmigen Vorsprung und eine Feder, die zwischen der Hülse und dem Vorsprung oder zwischen der Hülse und dem ringförmigen Vorsprung angeordnet ist. Diese Feder wirkt als Druckfeder oder als Zugfeder und fördert den Stift in Richtung der Ausnehmung zur Bildung einer Befestigung und eines Drehlagers für den Griff. Diese Anordnung ermöglicht einen sehr sicheren Betrieb des Griffes und eine einfache und verlässliche Handhabung.

Auch hat es sich bewährt, den Griff für einen Sterilisationsbehälter so weiterzubilden, dass der wenigstens eine Stift zweiteilig insbesondere zweiteilig und trennbar ausgebildet ist und dabei ein Teil des Stiftes ein Widerlager für die Feder bildet. Dies ermöglicht im Rahmen der Fertigung und Wartung eine sehr einfache Handhabung.

Nach einer besonders bevorzugten Ausführung sind wenigstens ein oder mehrere Schenkel plattenförmig ausgebildet und zur Aufnahme von Etiketten geeignet beziehungsweise dafür vorgesehen. Durch diese Ausbildung gelingt es, einerseits Etiketten zur Kennzeichnung der Sterilisationsbehälter oder des Inhalts der Sterilisationsbehälter gut lesbar und sicher und einfach mit dem Griff zu verbinden beziehungsweise an oder auf einem plattenförmigen Schenkel des Griffs anzuordnen, und andererseits den wenigstens einen Stift in einem plattenförmigen Schenkel sicher und damit gut handhabbar anzuordnen.

Dabei hat es sich besonders bewährt, diesen Griff so weiterzubilden, dass ein oder mehrere plattenförmige Schenkel eine oder mehrere Ausnehmungen zum Einschieben von Etiketten aufweisen. Dabei sind insbesondere mehrere Ausnehmungen nebeneinander und/oder hintereinander angeordnet. Durch das Vorsehen von wenigstens einer Ausnehmung zur Aufnahme einer oder mehrerer Etiketten gelingt es, die Handhabung einfach zu gestalten, indem durch Einschieben der Etiketten ein Austausch von Etiketten sehr einfach gestaltet werden kann. Durch die Anordnung von mehreren Ausnehmungen wird es möglich, mehrere differenzierte Etiketten für den Benutzer zur Verfügung zu stellen und mittels dieser mehreren Ausnehmungen diese mehreren Etiketten differenziert zu verwenden und dadurch die Handhabung vielfältiger und informativer und dadurch besser zu gestalten.

Dabei hat es sich bewährt, den Griff für einen Sterilisationsbehälter so weiterzubilden, dass verschiedene plattenförmige Schenkel eines oder mehrere Griffe an einem Sterilisationsbehälter unterschiedliche Farben aufweisen.

Dadurch gelingt es, die Zuordnung des Sterilisationsbehälters mit unterschiedlich farbigen, plattenförmigen Schenkeln sehr verlässlich zu gestalten und dadurch die Handhabung zu vereinfachen. Beispielsweise ist es möglich, Sterilisationsbehälter mit Griffen mit gelben plattenförmigen Schenkeln der Urologie in einem Krankenhaus zuzuordnen, während solche mit roten plattenförmigen Schenkeln der Kardiologie in einem Krankenhaus zugeordnet werden und dadurch sicher voneinander unterschieden werden können.

Daneben hat es sich auch bewährt, einen Griff für einen Sterilisationsbehälter dahingehend weiterzubilden, dass wenigstens ein horizontaler Steg einen runden insbesondere einen kreisrunden oder ovalen Querschnitt aufweist, wobei der Durchmesser eines Steges insbesondere wenigstens 1,5 cm gewählt ist. Dabei wird bevorzugt die Länge dieses Steges mit wenigstens 1,5 cm so gewählt, dass er wenigstens 10 cm beträgt. Diese Ausbildung eines Griffes erweist sich in der Handhabung beim Ergreifen des Griffes als besonders geeignet und gut handhabbar, da dadurch ein besonderes sicheres Ergreifen ermöglicht ist.

Ergänzend hat es sich auch bewährt, einen Griff für einen Sterilisationsbehälter so auszubilden, dass wenigstens eine insbesondere alle Ausnehmungen zur Aufnahme eines federnden Stiftes ein Sicherungselement insbesondere eine Sicherungsschraube zur Verhinderung eines unbeabsichtigten Entnehmens des Stiftes zugeordnet ist. Als Sicherungsschraube haben sich insbesondere Madenschrauben besonders bewährt, da sie vollständig in der Gewindeaufnahme aufgenommen werden und dadurch ein unerwünschtes Hängenbleiben an der Sicherungsschraube als Sicherungselement verhindert werden kann.

Ein bevorzugter Griff für einen Sterilisationsbehälter zeigt wenigstens eine wendelförmig ausgebildete Feder, die einen Stift umschließt. Durch diese Wahl der Feder ist es besonders einfach auf platzsparende und einfache Weise möglich, den Stift mit einer Federkraft zu beaufschlagen und eine Längsverschiebung des Stiftes in der Ausnehmung zur Aufnahme des Stiftes zu ermöglichen. Dies wirkt sich positiv auf die Größe des Griffes und damit auf die Handhabbarkeit des Griffes aus.

Eine bevorzugte Weiterbildung der Erfindung zeigt einen Sterilisationsbehälter mit wenigstens einem der vorgenannten beispielhaften, erfindungsgemäßen Griffe und mit einem wannenförmigen Unterteil, das mit wenigstens einer Aufnahmeplatte fest aber gegebenenfalls lösbar verbunden ist. Dabei weist wenigstens eine Aufnahmeplatte wenigstens eine Ausnehmung zur Aufnahme des wenigstens einen federnden Stiftes des Griffes und zur Bildung eines Drehlagers auf. Diese Aufnahmeplatte wird bevorzugt an einer Seitenwand des Unterteils befestigt, wobei dies insbesondere lösbar vorzugsweise mittels Verschraubungen erfolgt. Durch diese Ausbildung der Aufnahmeplatte mit daran angeordneten und integrierten Griff ist es möglich, diese Einheit vorzufertigen und auf einfache Weise zum endgültigen Sterilisationsbehälter zu verbinden.

Eine besonders bevorzugte Weiterbildung dieses Sterilisationsbehälters zeigt wenigstens eine Aufnahmeplatte mit mehreren Ausnehmungen zur Aufnahme jeweils eines federnden Stiftes des Griffes. Dabei sind mehrere Ausnehmungen und die zugeordneten federnden Stifte so orientiert, dass sie eine gemeinsame Drehachse für den Griff festlegen und die Ausnehmungen insbesondere an gegenüberliegenden Seiten einer Aufnahmeplatte angeordnet sind. Durch diese Anordnung gelingt es, die Handhabung des oder der Griffe sehr angenehm zu gestalten. Dies führt zu einer im besonderen Maße erhöhten Akzeptanz des Griffes für einen medizinischen Sterilisationsbehälter oder einen Sterilisationsbehälter mit wenigstens einem solchen Griff.

Die Erfindung wird nachfolgend anhand bevorzugter Ausführungsbeispiele unter Bezugnahme auf die Abbildungen beispielhaft erläutert. Die Erfindung ist nicht auf diese bevorzugten Ausführungsbeispiele beschränkt.
- Fig. 1: zeigt in einer schematischen Darstellung eine Seitenansicht eines beispielhaften, erfindungsgemäßen Sterilisationsbehälter mit beispielhaftem, erfindungsgemäßem Griff,
- Fig. 2: zeigt in einer schematischen Darstellung eine Seitenansicht eines anderen beispielhaften, erfindungsgemäßen Griffes,
- Fig. 3: zeigt eine schematische Darstellung der Rückansicht des beispielhaften, erfindungsgemäßen Griffes aus Fig. 2,
- Fig. 4: zeigt eine schematische Darstellung der Draufsicht des beispielhaften, erfindungsgemäßen Griffes aus Fig. 2 und
- Fig. 5: zeigt in schematischen Darstellungen des federbewehrten Stiftes des beispielhaften Griffes aus Fig. 2 in einer einem Zusammengebauten und einem zerlegten Zustand.

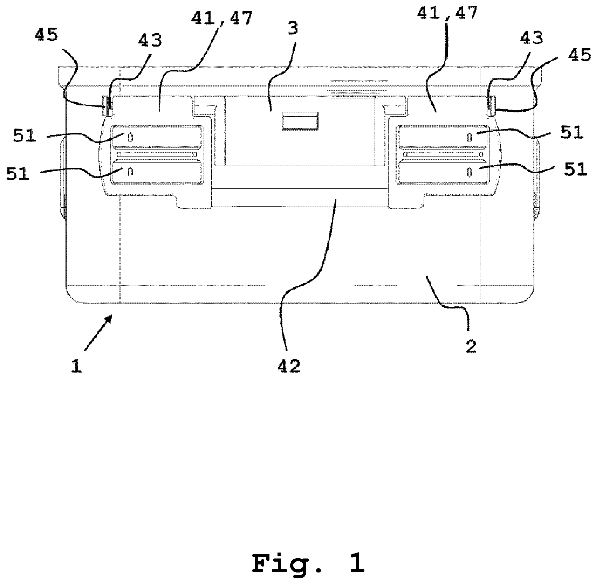

In Fig. 1 ist schematisch ein Sterilisationsbehälter 1 für medizinische Instrumente dargestellt. Dabei zeigt der Sterilisationsbehälter 1 ein wannenförmiges Unterteil 2 auf dem ein nicht dargestellter Deckel dichtend aufbringbar ist.

An der Seitenwand des Unterteils 2 ist eine Aufnahmeplatte 3 mit einem daran befestigten Griff 4 befestigt. Der Griff 4 ist U-förmig, bestehend aus zwei plattenförmigen Schenkeln 41,47 und aus einem horizontalen Steg 42, der zwischen den beiden plattenförmigen Schenkeln 41,47 angeordnet ist, ausgebildet. Der U-förmige Griff 4 ist mit der Aufnahmeplatte 3 mittels einer Befestigung des drehbar befestigt.

Die Befestigung erfolgt mithilfe von 2 Stiften 43, die federnd in den plattenförmigen Schenkeln 47 so gelagert sind, dass sie entlang ihrer Länge längsverschieblich geführt und von einer Feder 44 mit einer Federkraft so beaufschlagt sind, dass der Stift 43 durch die Federkraft der Feder 44 mit seinem, der Aufnahmeplatte 3 zugeordneten Ende in Ausnehmungen 31 in der Aufnahmeplatte 3 eingreifen. Durch dieses Eingreifen der Stifte 43 in die Aufnahmeplatte 3 ist eine Drehlagerung des U-förmigen Griffes 4 geschaffen, wobei die beiden Stifte 43 so orientiert sind, dass sie eine gemeinsame, einheitliche Drehachse definieren und dadurch ein sehr einfaches, reibungsarmes verschwenken des Griffes 4 um diese gemeinsame Drehachse ermöglicht ist.

Dabei sind die Stifte 43 in den plattenförmigen Schenkeln 47 so angeordnet und ausgebildet, dass sie gegen die Federkraft der jeweiligen Feder 44 so bewegt werden können, dass das Ende des Stiftes 43 aus der Ausnehmung 31 an der Aufnahmeplatte 3, die mit dem Wannen förmigen Unterteil 2. Sterilisationsbehälters 1 fest verbunden ist, heraus bewegt werden kann und dadurch der U-förmige Griff 4 von der Aufnahmeplatte 3 getrennt und entfernt werden kann. Dadurch ist es möglich, den U-förmigen Griff auf einfache Weise und damit gut handhabbar von dem Sterilisationsbehälter 1 zu lösen und damit getrennt von diesem zu reinigen, zu sterilisieren zu reparieren oder auch auf andere Art zu bearbeiten oder auch durch einen anderen Griff 4 zu ersetzen. Hierzu würde der angepasste oder andere U-förmige Griff 4 in eine Position an dem Sterilisationsbehälter mit der Aufnahmeplatte 3 so positioniert, dass die Stifte 43 Fluchten zu den Ausnehmungen 31 in der Aufnahmeplatte 3 sind und dann durch die Federkraft der Feder 44 jeder Stift 43 in die zugeordnete Ausnehmung 31 in der Aufnahmeplatte 3 eingreift und dadurch den neuen Griff 4 an der Aufnahmeplatte 3 drehbar befestigt.

Um die Stifte 43 zu betätigen weist das von der Aufnahmeplatte 3 abgewandelte Ende des Stiftes 43 einen Kopf 45 auf, der eine zylindrische Gestalt zeigt, welche sich lateral und ringförmig über den restlichen Stift 43 hinaus erstreckt. Dieser zylinderförmige Kopf 45 ermöglicht das sichere Ergreifen des Stiftes 43 und damit ein einfaches und verlässliches handhaben des beziehungsweise der Stifte 43 um den U-förmigen Griff 4 von der Aufnahmeplatte 3 des Sterilisationsbehälters 1 zu entnehmen oder an diesem zu befestigen. Der zylinderförmige Kopf 45 zeigt dabei einen Durchmesser der deutlich größer als der Schaft des Stiftes 43 ist aber zugleich etwa der Dicke der plattenförmigen Schenkel 47 entspricht. Dabei ist der zylinderförmige Kopf 45 so an dem Schaft des Stiftes 43 angeordnet, dass er im verbundenen Zustand von dem plattenförmigen Schenkel 47 beabstandet ist. Durch diese Beabstandung wird das sichere und verlässliche und einfache Ergreifen des Kopfes 45 ohne störende Behinderung durch den plattenförmigen Schenkel 47 ermöglicht.

Die beiden plattenförmigen Schenkel 47 zeigen jeweils 2 parallel zueinander verlaufende Ausnehmungen 51 für Etiketten. In diese Ausnehmungen 51 können bei Bedarf Etiketten 52 eingeführt werden, auf denen wesentliche Informationen zu dem Sterilisationsbehälter 1 beziehungsweise zu dem Inhalt des Sterilisationsbehälters 1 oder auch zu dessen Eigenschaften, wie beispielsweise Datum der letzten Sterilisierung oder Art oder Qualität der Sterilisierung, enthalten sein. Diese Information kann sowohl in alphanumerische Weise wie auch in farbcodierter Weise auf den Etiketten 52 oder auf dem U-förmigen Griff 4 insbesondere auf den Schenkeln 41,47 oder auf dem horizontalen Steg 42 angebracht sein.

Durch die drehbare Lagerung des U-förmigen Griffes 4 mithilfe der Stifte 43 in der Aufnahmeplatte 3 gelingt es, dass die Gewichtskraft des Griffes 4 den Griff bei einer Lage des Sterilisationsbehälters 1, wie sie in Fig. 1 dargestellt ist, nach unten verschwenkt ist und dadurch ein Anliegen des U-förmigen Griffes 4 an die Seitenwand des Wannen förmigen Unterteils 2 gegeben ist. Will ein Benutzer den Sterilisationsbehälter 1 ergreifen und wegtragen, so ergreift der Benutzer den U-förmigen Griff 4 an dem Steg 42 und verschwenkt diesen Steg 42 zusammen mit den diesen Steg 42 umschließenden plattenförmigen Schenkeln 47 um die durch die Stifte 43 definierte Drehachse, bis etwa eine horizontale Lage des U-förmigen Griffes 4 erreicht ist. In dieser Lage wird das weitere Verschwenken der Bewegung durch einen nicht dargestellten Anschlag begrenzt. In dieser Lage kann der Sterilisationsbehälter einfach und sicher endgültig angehoben und transportiert werden. Für den Transport und die Verwendung des Sterilisationsbehälters 1 sind die Informationen beispielsweise der vorgenannten Etiketten 52 von besonderer Bedeutung.

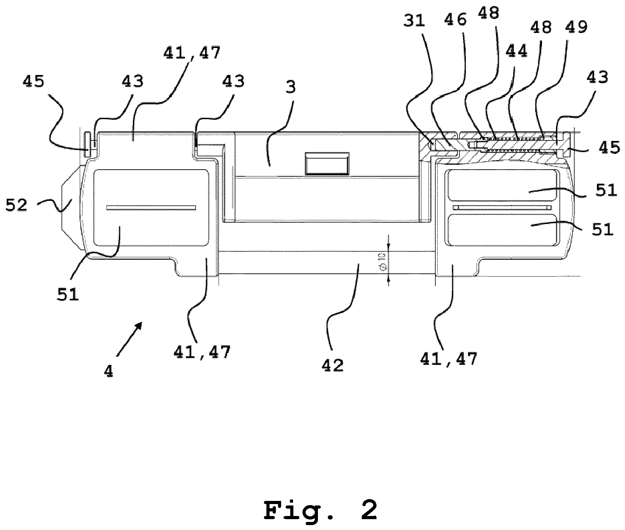

In Fig. 2 ist eine schematische Darstellung einer Seitenansicht eines anderen beispielhaften, erfindungsgemäßen Griffes 4 dargestellt. Dieser andere Griff 4 unterscheidet sich von dem in Fig. 1 dargestellten Griff 4 dadurch, dass die Ausnehmungen 51 für Etiketten 52 unterschiedlich ausgebildet sind. Der linke plattenförmige Schenkel 47 weist hier eine Ausnehmung 51 für ein großflächiges Etikett 52 auf. In diese Ausnehmung 51 ist von außen, seitlich ein Etikett 52 so eingeschoben, dass durch den großen sichtbaren Ausschnitt in der Ausnehmung 51 ein großer Teil des Etiketts 52 sichtbar geworden ist und darüber hinaus seitlich ein Ende des Etiketts 52 aus der Ausnehmung 51 herausragt. Dadurch ist es möglich, das Etikett 52 auf einfache Weise zu ergreifen und bei Bedarf aus der Ausnehmung 51 herauszuziehen und dadurch zu entnehmen beziehungsweise durch ein anderes Etikett 52 zu ersetzen.

Darüber hinaus zeigt die schematische Darstellung in Fig. 2 in einer teilweise geschnittenen Darstellung den inneren Aufbau des oberen Teils des rechten, plattenförmigen Schenkels 47 mit der Aufnahme für den Stift 43 , der im verbundenen Zustand mit seinem der Aufnahmeplatte 3 zugeordneten Ende 46, die als verjüngte Spitze mit einer angeschrägten Phase ausgebildet ist, in die Ausnehmung 31 an der Aufnahmeplatte 3 eingreift und dadurch ein Drehlager für den Griff 4 bildet. Der Stift 43 erstreckt sich von seinem Ende 46, das in die Ausnehmung 31 der Aufnahmeplatte 3 hineinragt, bis zu dem zylinderförmigen Kopf 45.

Der Stift 43 ist dabei aus zwei Teilen 43a, 43b gebildet, die ineinandergesteckt und fest miteinander verbunden sind. Das erste Teil 43a des Stiftes 43 zeigt das in die Ausnehmung 31 hineinragende Ende 46 und am anderen Ende eine Bohrung und einen dieses Ende umschließenden Vorsprung 50. Das zweite Teil 43b ist mit seinem vorderen Ende in eine Bohrung am Ende des ersten Teils 43a mit dem ringförmig umschließenden Vorsprung 50 eingebracht und auf diese Weise fest mit diesem verbunden. Das zweite Teil 43b ist von einer Feder 44, die als wendelförmige, auch spiralförmig genannte, Schraubenfeder ausgebildet ist, umschlossen und wird zusätzlich durch eine Hülse 49 , die ein Gleitlager für den zweiten Teil 43b und damit für den Stift 43 bildet, umschlossen und so geführt, dass der Stift 43 in der Hülse 49 in Längsrichtung des Stiftes 43 und damit in Richtung der Drehachse des Griffes 4 bewegt werden kann. Zwischen der Hülse und dem ringförmigen Vorsprung 50 ist die Feder 44 gespannt angeordnet. Da die Hülse 49 mittels einer Presspassung in der Aufnahme des plattenförmigen Schenkels 47 für den Stift 43 fest angeordnet ist und die Feder 44 unter Spannung steht, drückt die Feder 44 gegen den ringförmigen Vorsprung 50 und damit das erste Teil 43a in Richtung der Ausnehmung 31 in der Aufnahmeplatte 3 und damit auch gegen den ganzen Stift 43. Dementsprechend handelt es sich bei der Feder 44 um eine sogenannte Druckfeder.

Soll der Stift 43 aus der Ausnehmung 31 heraus bewegt oder entnommen werden, so wird der Stift 43 an dem Kopf 45 ergriffen und gegen die Federkraft der Druckfeder 44 nach rechts entlang der Drehachse beziehungsweise der Längsachse des Stiftes 43 so bewegt, dass der Abstand zwischen dem Kopf 45 und der Hülse 49 so lange vergrößert wird, bis das vordere Ende 46 des ersten Teils 43a und damit des Stiftes 43 aus der Ausnehmung 31 heraus bewegt ist. Dabei wird die Feder 44 weiter verkürzt und dadurch weiter gespannt. Wird der Kopf 45 losgelassen, so wird durch die Druckfeder 44 der Stift 43 so in Längsrichtung bewegt, dass der Abstand zwischen Kopf 45 und Hülse 49 wieder verkürzt wird und die Druckfeder 44 zumindest teilweise entspannt wird. Dadurch wird der Überstand des vorderen Endes 46 des ersten Teils 43a über den plattenförmigen Schenkel 47 vergrößert und, wenn der plattenförmige Schenkel 47 in geeigneter Position relativ zu der Aufnahmeplatte 3 angeordnet ist, das Ende 46 in die Ausnehmung 31 der Aufnahmeplatte 3 eingeführt. Durch das Vorsehen einer Phase am vorderen Ende 46 ist eine Spitze geschaffen, durch die das Einführen des Stiftes 43 in die Ausnehmung 31 aufgrund einer selbstzentrierenden Funktion der Spitze einfacher und sicherer erfolgen kann.

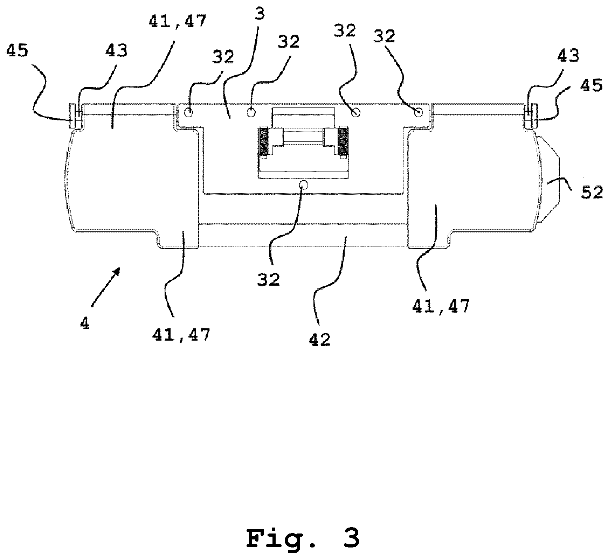

In Fig. 3 ist eine schematische Darstellung der Rückansicht des beispielhaften, erfindungsgemäßen Griffes aus Fig. 2 wiedergegeben. Die planen, unstrukturierten Rückseiten der plattenförmigen Schenkel 47 mit dem im Querschnitt kreisrunden horizontalen Steg 42 sind zu erkennen. Zwischen dem U-förmigen Griff 4 ist die Aufnahmeplatte 3 angeordnet und mittels der beiden Stifte 43 drehbar miteinander verbunden. Die Aufnahmeplatte 3 zeigt fünf Fixierelemente 32, die als Schrauben ausgebildet sind und dafür vorgesehen sind die Aufnahmeplatte 3 fest mit dem wannenförmigen Unterteil 2 des Sterilisationsbehälters 1 zu verbinden. Bei Bedarf kann diese Schraubverbindung mittels der Fixierelemente 32 wieder gelöst werden. In der Mitte der Aufnahmeplatte 3 ist ein Verschlussmechanismus angedeutet, der geeignet ist, den Deckel mit dem wannenförmigen Unterteil 2 zu einem vollständigen Sterilisationsbehälter 1 zu verbinden und diesen verschließend zu sichern.

Damit ist das wannenförmige Unterteil 2 über die Fixierelemente 32 mit der Aufnahmeplatte 3 fest verbunden und diese wiederum über die federnde und lösbare Verbindung durch den Aufbau und die Anordnung der federbewehrten Stifte 43 drehbar mit dem U-förmigen Griff 4 und damit mit den plattenförmigen Schenkeln 47 und dem horizontalen Steg 42 verbunden.

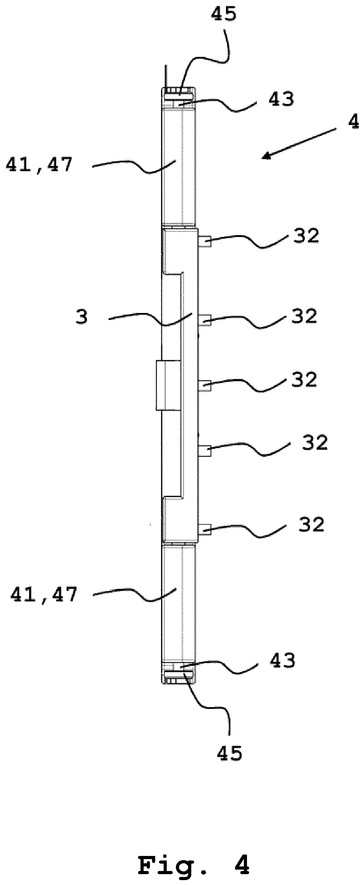

In Fig. 4 ist in schematischer Draufsicht von oben der in Fig. 2 und Fig. 3 dargestellte Griff 4 mit Aufnahmeplatte 3 dargestellt. Die Fixierelemente 32 ragen deutlich aus der Aufnahmeplatte 3 heraus und ermöglichen dadurch eine feste Verbindung zu einem wannenförmigen Unterteil 2 des Sterilisationsbehälters 1. Die zylinderförmigen Köpfe 45 der Stifte 43 zeigen einen Durchmesser, der etwas geringer als die Stärke der plattenförmigen Schenkel 47 beziehungsweise die maximale Stärke der Aufnahmeplatte 3 ist. Dennoch ist der Durchmesser des zylinderförmigen Kopfes 45 mehr als doppelt so groß wie der Durchmesser des Schaftes des Stiftes 43 beziehungsweise dessen Teile 43a, 43b. Dadurch ist eine gute Handhabung zum Verriegeln oder Entriegeln der Stifte 43 in den Ausnehmungen 31 der Aufnahmeplatte 3 gegeben.

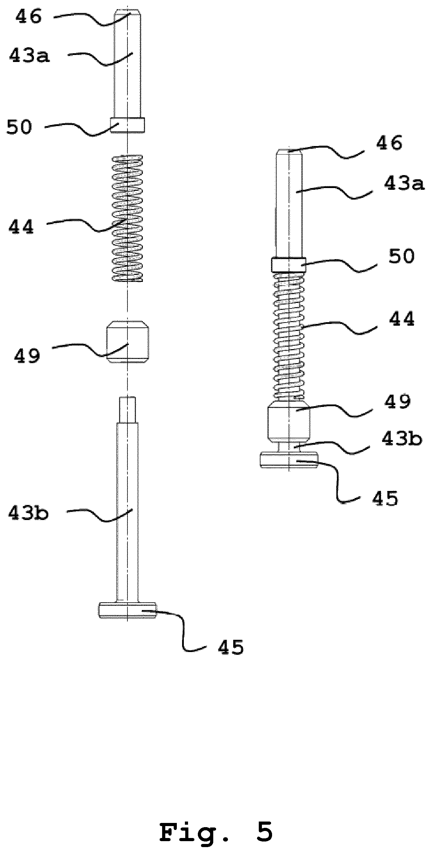

In Fig. 5 ist der schematische Aufbau eines Stiftes 43 aus den Fig. 1-4 mit einer Hülse 49 und der Feder 44 einmal als zusammengebaute Einheit und einmal zerlegt dargestellt.

Der Stift 43 ist zweiteilig aufgebaut und besteht aus einem ersten Teil 43a und einem zweiten Teil 43b.

Das erste Teil 43a des Stiftes 43 zeigt ein Ende 46, das in die Ausnehmung 31 hineinragen soll, und eine Bohrung am anderen Ende sowie einen dieses Ende umschließenden ringförmigen Vorsprung 50.

Das zweite Teil 43b ist mit seinem vorderen verjüngten Ende so ausgebildet, dass dieses Ende in die Bohrung des ersten Teils 43a mit dem ringförmig umschließenden Vorsprung 50 eingebracht und auf diese Weise fest mit diesem verbunden werden kann.

Das zweite Teil 43b ist im verbundenen, zusammengebauten Zustand von einer Feder 44, die als wendelförmige oder auch spiralförmige Schraubenfeder ausgebildet ist, umschlossen. Zusätzlich ist der Schaft des zweiten Teil 43b im verbundenen, zusammengebauten Zustand von einer Hülse 49, die ein Gleitlager für den zweiten Teil 43b und damit für den Stift 43 bildet, umschlossen. Dabei ist die Anordnung aus Feder 44, aus Hülse 49 und ringförmigen Vorsprung 50 so realisiert, dass die als Druckfeder ausgebildete Feder 44 bestrebt ist, die Hülse 49 und den ringförmigen Vorsprung 50 möglichst weit auseinanderzudrücken. Dies ist möglich, da der Stift 43 in der Hülse 49 in Längsrichtung des Stiftes 43 bewegt werden kann, was durch die Ausbildung der Hülse 49 als Gleitlager für den Stift 43 sehr reibungsarm ermöglicht ist.

Nachdem die in Fig. 5 dargestellten Komponenten zu einer zusammengebauten Einheit im Rahmen eines Vormontageprozesses verbunden sind, kann diese Einheit in die Aufnahme für den Stift 43 in dem plattenförmigen Schenkel 47 im Bereich des Endes, das von dem Steg 42 abgewandt ist, so eingebracht werden, dass das Ende 46 des Stiftes 43 , welches in die Ausnehmung 31 an der Aufnahmeplatte 3 hineinreichen soll, durch die Aufnahme hindurch reicht und die Hülse 49 mittels Presspassung fest in die Aufnahme eingepresst und mit dieser Form schlüssig verbunden ist. Dadurch ist ein sehr sicherer Aufbau des Griffes 4 mit den federbewehrten Stiften 43 in den plattenförmigen Schenkeln 47 gegeben, der sich zudem durch eine einfache und verlässliche Fertigung auszeichnet. Damit ist auch eine qualitativ hochwertige Handhabung des Griffes 4 für einen medizinischen Sterilisationsbehälter 1 gewährleistet.

### Bezugszeichenliste

- 1: Sterilisationsbehälter
- 2: Wannenförmiges Unterteil
- 3: Aufnahmeplatte
- 31: Ausnehmung in Aufnahmeplatte für Stift
- 32: Fixierelement für Aufnahmeplatte am Unterteil
- 4: Griff
- 41: Schenkel
- 42: Horizontaler Steg
- 43: Stift
- 43a: Erster Teil des zweiteiligen Stiftes
- 43b: Zweiter Teil des zweiteiligen Stiftes
- 44: Feder
- 45: Griffelement, zylinderförmiger Kopf des Stiftes
- 46: Spitze des Stiftes
- 47: Plattenförmiger Schenkel
- 48: Aufnahme für Stift
- 49: Hülse
- 50: Ringförmiger Vorsprung an Stift
- 51: Ausnehmung für Etiketten
- 52: Etikett

## Patentansprüche

1. Griff (4) für einen Sterilisationsbehälter (1), wobei der Griff (4) U-förmig mit zwei Schenkeln (41) und einem ein Halteelement bildender horizontaler Steg (42), der die beiden Schenkel (41) miteinander verbindet, ausgebildet ist,
wobei der Griff (4) mittels einer Befestigung am Sterilisationsbehälter (1) drehbar befestigbar ist, **dadurch gekennzeichnet,**
**dass** die Befestigung wenigstens einen Stift (43) aufweist, der in eine Ausnehmung (31) am Sterilisationsbehälter (1) eingreifen kann, der ein Drehlager bilden kann und der aus der Ausnehmung (31) entgegen einer Federkraft einer Feder (44) entnommen werden kann.

2. Griff für einen Sterilisationsbehälter nach Anspruch 1, wobei die Befestigung mehrere federnd gelagerte Stifte (43) aufweist, die jeweils in eine Ausnehmung (31) am Sterilisationsbehälter (1) eingreifen können und ein Drehlager bilden können und aus der Ausnehmung (31) entgegen der Federkraft einer Feder (44) entnommen werden können und wobei mehrere der Stifte (43) so orientiert sind, dass sie eine gemeinsame Drehachse für den Griff (4) festlegen.

3. Griff für einen Sterilisationsbehälter nach einem der Ansprüche 1 oder 2, wobei wenigstens ein federnd gelagerter Stift (43) eine verjüngende Spitze (46) aufweist, welche geeignet ist, in die Ausnehmung (31) einzugreifen.

4. Griff für einen Sterilisationsbehälter nach einem der Ansprüche 1 bis 3, wobei wenigstens ein federnd gelagerter Stift (43) ein Griffelement (45) aufweist, das den Stift (43) lateral überragt und das insbesondere an einem Ende des Stiftes (43) angeordnet ist und einen Kopf (45) oder einen zylinderförmigen Kopf (45) bildet.

5. Griff für einen Sterilisationsbehälter nach einem der Ansprüche 1 bis 4, wobei wenigstens ein Stift (43) in einer einen Schenkel (41) querenden Aufnahme (48) geführt ist und insbesondere die Aufnahme (48) einen Stift (43) umschließt und ein Gleitlager für einen darin angeordneten Stift (43) bildet.

6. Griff für einen Sterilisationsbehälter nach einem der vorstehenden Ansprüche, wobei eine Hülse (49) mit einem Schenkel (41) verbunden ist und eine Führung und/oder ein Gleitlager für einen darin angeordneten Stift (43) bildet.

7. Griff für einen Sterilisationsbehälter nach Anspruch 6, wobei die Hülse (49) in der Aufnahme (48) angeordnet ist und insbesondere die Aufnahme (48) abschließt.

8. Griff für einen Sterilisationsbehälter nach einem der vorstehenden Ansprüche 5 bis 6, wobei wenigstens eine Feder zwischen dem Stift (43) und der Aufnahme (48) in dem Schenkel (41,47) angeordnet ist und als Druckfeder oder als Zugfeder ausgebildet ist.

9. Griff für einen Sterilisationsbehälter nach Anspruch 8, wobei ein Stift (43) einen Vorsprung oder ringförmigen Vorsprung (50) aufweist und die Feder (44) zwischen der Hülse (49) und dem Vorsprung oder zwischen der Hülse (49) und dem ringförmigen Vorsprung (50) angeordnet ist und als Druckfeder oder als Zugfeder ausgebildet ist.

10. Griff für einen Sterilisationsbehälter nach einem der Ansprüche 1 bis 9, wobei wenigstens ein Stift (43) zweiteilig insbesondere zweiteilig und trennbar ausgebildet ist und ein Teil (43a) des Stiftes (43) ein Widerlager für die Feder (44) bildet.

11. Griff für einen Sterilisationsbehälter nach einem der vorstehenden Ansprüche, wobei ein oder mehrere Schenkel (41,47) plattenförmig ausgebildet ist oder sind und zur Aufnahme von Etiketten (52) geeignet und vorgesehen ist oder sind.

12. Griff für einen Sterilisationsbehälter nach einem der vorstehenden Ansprüche, wobei wenigstens einer Ausnehmung (31) zur Aufnahme eines federnden Stiftes (43) eine Sicherungsschraube zur Verhinderung eines unbeabsichtigten Entnehmens des Stiftes (43) zugeordnet ist.

13. Griff für einen Sterilisationsbehälter nach einem der vorstehenden Ansprüche, wobei wenigstens eine Feder (44) wendelförmig ausgebildet ist und einen Stift (43) umschließt.

14. Sterilisationsbehälter (1) mit wenigstens einem Griff (4) nach einem der vorstehenden Ansprüche, mit einem wannenförmigen Unterteil (2), das mit wenigstens einer Aufnahmeplatte (3) fest verbunden ist, wobei die Aufnahmeplatte (3) wenigstens eine Ausnehmung (31) zur Aufnahme des wenigstens einen federnden Stiftes (43) des Griffes (4) aufweist.

15. Sterilisationsbehälter nach Anspruch 14, mit einem wannenförmigen Unterteil (2), das mit wenigstens einer Aufnahmeplatte (3) fest verbunden ist, wobei die Aufnahmeplatte (3) mehrere Ausnehmungen (31) zur Aufnahme jeweils eines federnden Stiftes (43) des Griffes (4) aufweist, wobei mehrere Ausnehmungen (31) und die zugeordneten federnden Stifte (43) so orientiert sind, dass sie eine gemeinsame Drehachse für den Griff (4) festlegen und die Ausnehmungen (31) insbesondere an gegenüberliegenden Seiten einer Aufnahmeplatte (3) angeordnet sind.
